# EUROPÄISCHE PATENTSCHRIFT

(11) **EP 2 193 139 B1**
(45) Veröffentlichungstag und Bekanntmachung des Hinweises auf die Patenterteilung: **08.05.2013**
(21) Anmeldenummer: 08804292.4
(22) Anmeldetag: 17.09.2008
(51) Int. Cl.: C07H 1/00, C07H 21/00

(54) **VERFAHREN ZUR FLÜSSIGPHASENSYNTHESE EINES POLYMERS**
METHOD FOR FLUID-PHASE SYNTHESIS OF A POLYMER
PROCÉDÉ DE SYNTHÈSE EN PHASE LIQUIDE D'UN POLYMÈRE

(30) Priorität: 19.09.2007 DE 102007044765
(43) Veröffentlichungstag der Anmeldung: 09.06.2010
(73) Patentinhaber: Secutech International Pte. Ltd., Singapore 069443 (SG)
(72) Erfinder: WOLFRUM, Christian, 91052 Erlangen (DE); PRINZ, Wolfgang, A-4240 Freistadt (AT)
(74) Vertreter: Ehnis, Tobias
(86) Internationale Anmeldenummer: PCT/EP2008/062337
(87) Internationale Veröffentlichungsnummer: WO 2009/037268

(56) Entgegenhaltungen:
- BONORA G M ET AL: "Large scale, liquid phase synthesis of oligonucleotides by the phosphoramidite approach" NUCLEIC ACIDS RESEARCH, OXFORD UNIVERSITY PRESS, SURREY, GB, Bd. 21, Nr. 5, 1. Januar 1993 (1993-01-01), Seiten 1213-1217, XP001537118 ISSN: 0305-1048 in der Anmeldung erwähnt

## Beschreibung

Die Erfindung betrifft ein Verfahren zur Flüssigphasensynthese eines aus n Monomeren gebildeten Polymers.

Aus dem Stand der Technik ist es bekannt, Desoxyribonukleinsäurepolymere zu synthetisieren, indem an ein Nukleotid, welches mit seinem 3'-Ende an einem festen Träger immobilisiert ist, schrittweise jeweils am vorhandenen oder neu entstehenden 5'-Ende ein Nukleotid angehängt wird, bis ein Oligo- oder Polynukleotid der gewünschten Länge gebildet ist. Dieses Oligo- oder Polynukleotid wird dann vom festen Träger abgespalten. Der feste Träger liegt dabei üblicherweise in einer Chromatografie-Säule vor. Je größer die dabei verwendete Säulenpackung ist, desto schwerer ist das Verfahren handhabbar. Insbesondere ist eine homogene Packung der Säule schwer zu erreichen und wegen des bei großen Säulen über die Laufstrecke auftretenden Druckverlusts ist eine leistungsfähige Pumpe erforderlich. Problematisch ist außerdem der bei Säulen stets auftretende Randeffekt, der dazu führt, dass eine chromatografische Lauffront in der Nähe der Wandung der Säule langsamer wandert als in der Mitte der Säule und der eine verringerte Effizienz des Verfahrens bewirkt.

Als alternatives Verfahren zur Synthese großer DNA Mengen ist aus Bonora, G. M. et al., Nucleic Acids Research, 1993, Band 21, Nr. 5, Seiten 1213 bis 1217 ein Verfahren zur Flüssigphasensynthese von Oligonukleotiden bekannt, bei welchem Polyethylenglycol (PEG) als löslicher Träger und Phosphoramidit-Derviate als Synthone verwendet werden. Dabei ist am 3'-Ende eines bei der Synthesereaktion zu verlängernden Nukleosids Polyethylenglycol gebunden. Nach Entfernen einer an dem Nukleosid vorhandenen Dimethoxytrityl-Schutzgruppe erfolgt am 5'-Ende des Nukleosids eine Kondensation mit einem Nukleosid-Phosphoramidit. Das Reaktionsprodukt wird präzipitiert und rekristallisiert. Nach Durchführen einer Cappingreaktion und einer nachfolgenden Oxidationsreaktion wird das erhaltene PEG-Phosphat-Derivat präzipitiert, filtriert und rekristallisiert. Anschließend werden die genannten Verfahrensschritte der Detritylierung, Kondensation, Präzipitation, Rekristallisation, Capping-Reaktion, Oxidationsreaktion, Präzipitation, Filtration und Rekristallisation solange wiederholt, bis das Polymer die gewünschte Länge aufweist. Abschließend erfolgt eine Abspaltung des Polyethylenglycols und eine Fällung des erhaltenen Oligonukleotids. Das Oligonukleotid wird anschließend gelöst und durch Ionenaustauscherchromatografie gereinigt.

Nachteilig bei beiden genannten Verfahren ist, dass bei zunehmender Kettenlänge die Wahrscheinlichkeit der Herstellung eines fehlerhaften Polynukleotids exponentiell zunimmt. Der Grund dafür besteht darin, dass bei jedem Kopplungs- bzw. Kondensationsschritt ein geringer Prozentsatz der zu verlängernden Nukleotidketten nicht reagiert. Dies führt dazu, dass bei einer angenommenen Kopplungseffizienz von 98% bei einem aus nur 10 Nukleotiden gebildeten Oligomer bereits 18,3% fehlerhafte Oligomere entstehen. Bei einem aus 100 Nukleotiden gebildeten Polymer sind bereits 86,7% der gebildeten Polymere fehlerhaft. Die fehlerhaften Polymere können die korrekt synthetisierten Polymere mengenmäßig also bei weitem übersteigen. Das führt dazu, dass ein hoher Aufwand betrieben werden muss, um die fehlerhaften Polymere, beispielsweise mittels Chromatografie, abzutrennen, um das gewünschte Polymer in reiner Form zu erhalten. Die Herstellung eines gewünschten Polymers ist daher wegen der erforderlichen Reinigung und der großen Menge der für die Herstellung fehlerhafter Polymere zwangsläufig zu verwendenden Reagenzien relativ kostenaufwändig.

Aufgabe der vorliegenden Erfindung ist es, ein Verfahren zur Flüssigphasensynthese eines aus Monomeren gebildeten Polymers bereitzustellen, mittels welchem das Polymer kostengünstig in großer Menge und in hoher Reinheit hergestellt werden kann.

Diese Aufgabe wird durch die Merkmale des Anspruchs 1 gelöst. Zweckmäßige Ausgestaltungen der Erfindung ergeben sich aus den Merkmalen der Ansprüche 2 bis 15.

Erfindungsgemäß ist ein Verfahren zur Flüssigphasensynthese eines aus n Monomeren gebildeten ersten Polymers mit folgenden Schritten vorgesehen:
a) Bereitstellen einer gelösten ersten Verbindung, wobei die erste Verbindung aus einem ersten Monomer und einem eine erste spezifische Löslichkeit aufweisenden zweiten Polymer gebildet ist, wobei das erste Monomer und das zweite Polymer durch eine erste Bindung miteinander verbunden sind, wobei die erste Bindung durch eine erste Spaltungsreaktion gespalten werden kann, wobei das erste Monomer eine aktive oder eine aktivierbare erste Gruppe aufweist,
b) Bereitstellen einer gelösten zweiten Verbindung, wobei die zweite Verbindung aus einem zweiten Monomer und einem eine zweite spezifische Löslichkeit aufweisenden dritten Polymer gebildet ist, wobei das zweite Monomer und das dritte Polymer durch eine zweite Bindung miteinander verbunden sind, wobei die zweite Bindung durch eine zweite Spaltungsreaktion gespalten werden kann, wobei das zweite Monomer eine aktive oder eine aktivierbare zweite Gruppe aufweist, welche durch eine Verknüpfungsreaktion mit der ersten Gruppe unter Ausbildung einer dritten Bindung zwischen dem ersten und dem zweiten Monomer reagieren kann, wobei die erste und die zweite spezifische Löslichkeit verschieden sind,
c) Inkontaktbringen der ersten und der zweiten Verbindung, so dass die Verknüpfungsreaktion erfolgt, wobei im Falle des Vorliegens der aktivierbaren ersten und/oder der aktivierbaren zweiten Gruppe eine Aktivierung dieser ersten und/oder zweiten Gruppe durch Zusatz eines Aktivators erfolgt,
d) Durchführen einer für das zweite oder dritte Polymer spezifischen Fällungsreaktion und Absondern dabei gefällter erster oder zweiter Verbindung und des bei Schritt lit. c) gebildeten Reaktionsprodukts,
e1) Lösen der abgesonderten ersten oder zweiten Verbindung und des Reaktionsprodukts in einem Lösungsmittel, Durchführen einer für dasjenige zweite oder dritte Polymer spezifischen Fällungsreaktion für das die bei Schritt lit. d) durchgeführte Fällungsreaktion nicht spezifisch war, Absondern dabei gefällter Reaktionsprodukte und Lösen des Reaktionsprodukts in dem Lösungsmittel oder einem weiteren Lösungsmittel,
   oder
e2) Inkontaktbringen der abgesonderten ersten oder zweiten Verbindung und des Reaktionsprodukts mit einem Lösungsmittel, in welchem sich im Wesentlichen nur das Reaktionsprodukt löst, in welchem sich aber im Wesentlichen nicht die bei Schritt lit. d) gefällte erste oder zweite Verbindung löst,
f) Durchführen der zweiten Spaltungsreaktion mit dem Reaktionsprodukt, wobei ein weiteres Reaktionsprodukt und das dritte Polymer gebildet werden, wobei an dem weiteren Reaktionsprodukt die erste aktive Gruppe gebildet wird und
g) (n-2)-faches Wiederholen der Schritte lit. b) bis lit. f), wobei an Stelle der ersten Verbindung das bei Schritt lit. f) gebildete weitere Reaktionsprodukt tritt und an Stelle der zweiten Verbindung jeweils eine weitere Verbindung tritt, wobei die weitere Verbindung aus einem weiteren Monomer und dem dritten Polymer gebildet ist.

Das weitere Monomer und das zweite Monomer können verschieden oder identisch sein. Bei dem ersten Polymer kann es sich auch um ein Oligomer handeln. Unter einer für das zweite Polymer spezifischen Fällungsreaktion wird eine Fällungsreaktion verstanden, bei welcher das dritte Polymer im Wesentlichen in Lösung bleibt. Unter einer für das dritte Polymer spezifischen Fällungsreaktion wird eine Fällungsreaktion verstanden, bei welcher das zweite Polymer im Wesentlichen in Lösung bleibt. Es versteht sich, dass bei den Reaktionspartnern und dem Reaktionsprodukt sowie dem weiteren Reaktionsprodukt nicht nur ein Molekül, sondern eine Vielzahl von Molekülen des gleichen Typs gemeint sind, auch wenn zur Bezeichnung der Moleküle eine Singularform verwendet wird. So werden beispielsweise eine Vielzahl identischer erster Verbindungen und eine Vielzahl identischer zweiter Verbindungen bereitstellt und es entsteht eine Vielzahl identischer Reaktionsprodukte bei Durchführung des Schritts lit. c).

Das wesentliche Merkmal des erfindungsgemäßen Verfahrens besteht darin, dass das sich bildende erste Polymer durch das daran gebundene jeweils spezifisch fällbare zweite und dritte Polymer nach jeder Verknüpfungsreaktion auf einfache Weise von nicht-reagiertem Monomer und von nicht-verlängertem weiteren Reaktionsprodukt abgetrennt wird, so dass eine aufwändige Reinigung am Ende der Synthese entfällt. Präzipitate können einfach durch Zentrifugieren oder Filtrieren abgetrennt werden. Dies ist im Gegensatz zu einer chromatografischen Trennung auch in großem Maßstab kostengünstig möglich. Weiterhin wird durch das erfindungsgemäße Verfahren bewirkt, dass sich fehlerhafte Reaktionsprodukte nicht anreichern und es somit bei zunehmender Kettenlänge nicht wie bei der herkömmlichen Festphasensynthese zu einer exponentiellen Zunahme fehlerhafter Polymere kommt. Durch das Entfallen einer aufwändigen Reinigung und das Verhindern der Entstehung fehlerhafter Polymere in großer Menge sind die Herstellungskosten des Polymers deutlich niedriger als bei einem mit herkömmlichen Verfahren hergestellten Polymer.

Für die Durchführung der Fällungsreaktionen kommen verschiedene Möglichkeiten in Betracht. So kann die Löslichkeit des zweiten oder dritten Polymers durch Zugabe eines Lösungsmittels, in welchem das entsprechende Polymer unlöslich ist, verringert werden. Beispielsweise kann Polyethylenglycol (PEG) mit Hilfe von Methyl-tertiär-butylether (MtBE) ausgefällt werden. Weiterhin kann zum Fällen die "low critical solution temperature" ausgenutzt werden. Dabei wird durch Temperaturerhöhung in einem entsprechenden Medium ein Ausfällen bewirkt. Beispielsweise kann in Wasser gelöstes Poly(N-iso-propylacrylamid) bei Temperaturen über 30,2°C ausgefällt werden. Es besteht auch die Möglichkeit die "upper critical solution temperature" auszunutzen. Dabei wird das Ausfällen durch eine Temperatursenkung in einem entsprechenden Medium bewirkt. Beispielsweise fällt in Azeton gelöstes PEG bei Temperaturen unterhalb von 30°C aus. Weiterhin können Polymere mittels eines chemischen Fällungsmittel gefällt werden, sofern das Ausfällen reversibel ist, da das Präzipitat für weitere Kopplungsschritte wieder gelöst werden muss. Beispielsweise können Organobariumverbindungen als Polymere in Form von Sulfaten gefällt werden.

Bei einer Oligonukleotidsynthese besteht ein weiterer Vorteil des erfindungsgemäßen Verfahrens darin, dass ein bei der herkömmlichen Oligonukleotidsynthese erforderlicher Capping-Schritt nicht erforderlich ist. Der Capping-Schritt wird bei herkömmlichen Verfahren durchgeführt, um OH-Gruppen am 5'-C-Atom, welche bei der Kondensation nicht reagiert haben, beispielsweise durch Acetylierung mittels Essigsäureanhydrid, zu blockieren und dadurch von einer weiteren Reaktion auszuschließen. Da das zur Blockierung verwendete Mittel jedoch bei der weiteren Reaktion stört, ist nach dem Capping ein weiterer Reinigungsschritt erforderlich. Auch dieser entfällt beim erfindungsgemäßen Verfahren. Das Capping ist beim erfindungsgemäßen Verfahren deshalb nicht erforderlich, weil beim Schritt lit. e1) nur das Reaktionsprodukt gefällt wird bzw. beim Schritt lit. e2) nur das Reaktionsprodukt in Lösung geht. Dadurch verbleiben keine Nukleotide, welche zuvor nicht reagiert haben und freie OH-Gruppen aufweisen im Reaktionsansatz. Für den Fall, dass bei der spezifischen Fällungsreaktion gemäß Schritt lit. e1) bzw. beim spezifischen Lösen gemäß Schritt lit. e2) nicht-reagierte Nukleotide in geringen Mengen mitgeschleppt werden sollten, kann jedoch zur Erhöhung der Reinheit des Reaktionsprodukts ein zusätzlicher Capping-Schritt durchgeführt werden.

Bei einer bevorzugten Ausgestaltung des Verfahrens wird nach Schritt lit. g) oder zwischen den Schritten lit. f) und lit. g) eine für das zweite Polymer spezifische Fällungsreaktion durchgeführt und das dabei gefällte bei Schritt lit. f) gebildete weitere Reaktionsprodukt abgesondert. Alternativ kann zwischen den Schritten lit. f) und lit. g) eine für das dritte Polymer spezifische Fällung durchgeführt und das dabei gefällte, bei Schritt lit. f) gebildete dritte Polymer abgesondert werden. Durch das Durchführen der Fällung zwischen den Schritten lit. f) und lit. g) kann eine noch höhere Reinheit des Endprodukts erzielt werden, weil das nach Durchführung des Schritts lit. f) eine Verunreinigung darstellende dritte Polymer nicht in einen weiteren Reaktionsschritt mitgeschleppt wird.

Vorzugsweise handelt es sich bei dem ersten und dem zweiten Monomer jeweils um ein Nukleotid. Da Oligo- und Polynukleotide zunehmend zur Markierung von Produkten verwendet werden, besteht hierfür ein großer Bedarf, der mit herkömmlicher Technik nur mit hohem Kostenaufwand zu decken ist. Der verhältnismäßig geringe Reinigungsaufwand bei dem erfindungsgemäßen Verfahren macht sich bei der Herstellung von Oligo- und Polynukleotiden besonders bemerkbar. 50% der Herstellungskosten eines mit herkömmlicher Technik hergestellten Oligo- oder Polynukleotids entfallen auf die Kosten der chromatografischen Reinigung des Produkts.

Die aktivierbare erste Gruppe kann aus einem am 5'-C-Atom des Nukleotids gebundenen Sauerstoffatom und einer daran gebundenen Schutzgruppe, insbesondere einer Dimethoxytritylgruppe, bestehen. Bei der Schutzgruppe kann es sich auch um das dritte Polymer handeln, wobei es besonders bevorzugt ist, wenn das dritte Polymer an eine Dimethoxytritylgruppe gebunden ist. Die Aktivierung der aktivierbaren ersten Gruppe kann durch eine Abspaltung der Schutzgruppe unter Reduktion des Sauerstoffatoms, insbesondere durch eine organische Säure als Aktivator, erfolgen. Dadurch kann beispielsweise eine OH-Gruppe als aktive Gruppe gebildet werden. Bei der organischen Säure kann es sich um eine schwache organische Säure, wie z. B. Dichloressigsäure, handeln. Handelt es sich bei der Schutzgruppe um eine Dimethoxytritylgruppe, wird die Abspaltung als Detritylierung bezeichnet.

Bei der aktiven ersten Gruppe kann es sich um eine am 5'-C-Atom des das erste Monomer bildenden Nukleotids angeordnete OH-Gruppe handeln. Das zweite Monomer kann ein Nukleosid-Phosphoramidit sein. Vorzugsweise wird das Nukleosid-Phosphoamidit vor dem oder beim Inkontaktbringen gemäß Schritt lit. c), insbesondere mittels H-Tetrazol, Ethylthiotetrazol oder einem anderen Tetrazolderivat oder Dicyanoimidazol, durch Protonierung aktiviert.

Bei Verwendung von Phosphoramidit kann zwischen den Schritten lit. c) und lit. g) ein Oxidationsschritt zur Oxidation des gebildeten Phosphittriesters zum Phosphotriester, insbesondere mittels Jod als Oxidationsmittel, durchgeführt werden. Dadurch kann verhindert werden, dass Phosphoramidit bei einer nachfolgenden Detritylierung abgespalten wird. Das Oxidationsmittel wird dabei vorzugsweise so gewählt, dass dadurch auch eine ggf. vorhandene Bindung zur Dimethoxytritylgruppe durch eine Oxidationsreaktion gespalten wird.

Vorzugsweise ist das erste Monomer über ein Sauerstoffatom, insbesondere innerhalb einer Etherbindung oder einer Esterbindung, an das zweite Polymer gebunden. Die erste Verbindung kann bei der Verknüpfungsreaktion über ein weiteres Sauerstoffatom, insbesondere innerhalb einer Etherbindung oder einer Esterbindung, mit der zweiten Verbindung verknüpft werden. Nach dem (n-2)-fachen Wiederholen der Schritte lit. b) bis lit. f) kann die erste Spaltungsreaktion durchgeführt werden, um das gebildete erste Polymer von dem zweiten Polymer zu trennen. Dies ist dann nicht erforderlich, wenn das Verbundensein des zweiten Polymers mit dem ersten Polymer bei der weiteren Verwendung des ersten Polymers nicht stört oder dabei sogar nützlich ist. Das kann insbesondere dann der Fall sein, wenn das gebildete Polymer ein als Fänger-Molekül dienendes Polynukleotid ist und die damit hybridisierenden Ziel-Polynukleotide durch eine spezifische Reaktion gefällt werden sollen.

Bei Durchführung der ersten Spaltungsreaktion kann eine Reduktion des gebildeten Phosphotriesters erfolgen. Die erste Spaltungsreaktion kann beispielsweise mittels einer Ammoniumhydroxid-Lösung durchgeführt werden.

Bei einer bevorzugten Ausgestaltung des erfindungsgemäßen Verfahrens handelt es sich bei dem zweiten oder dem dritten Polymer um Polyethylenglycol. Es kann sich bei dem dritten oder zweiten Polymer auch um Polystyrol handeln. In jedem Fall sind aber das zweite und das dritte Polymer verschieden.

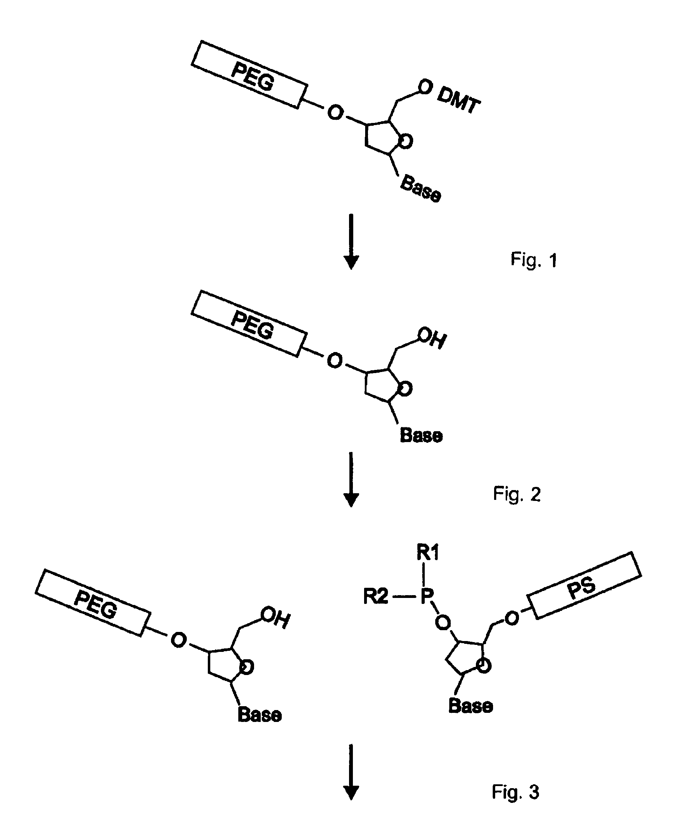

Nachfolgend wird die Erfindung anhand der schematischen Darstellung in den Figuren 1 bis 11 und eines Ausführungsbeispiels näher erläutert.

Bei der gesamten nachfolgend beschriebenen Reaktion wird Acetonitril als Lösungsmittel verwendet. Fig. 1 zeigt ein an dessen 3'-C-Atom über ein Sauerstoffatom an eine Polyethylenglycol(PEG)-Gruppe gebundenes Nukleosid. Das Nukleosid weist an seinem 5'-C-Atom eine über ein weiteres Sauerstoffatom gebundene Dimethoxytrityl(DMT)-Schutzgruppe auf. Das 5'-Sauerstoffatom mit der DMT-Schutzgruppe stellt eine aktivierbare Gruppe dar. Die DMT-Schutzgruppe wird mittels einer schwachen organischen Säure, wie beispielsweise Dichloressigsäure, abgespalten, so dass eine freie OH-Gruppe als aktive Gruppe gebildet wird. Das entsprechende Reaktionsprodukt ist als erste Verbindung in Fig. 2 dargestellt.

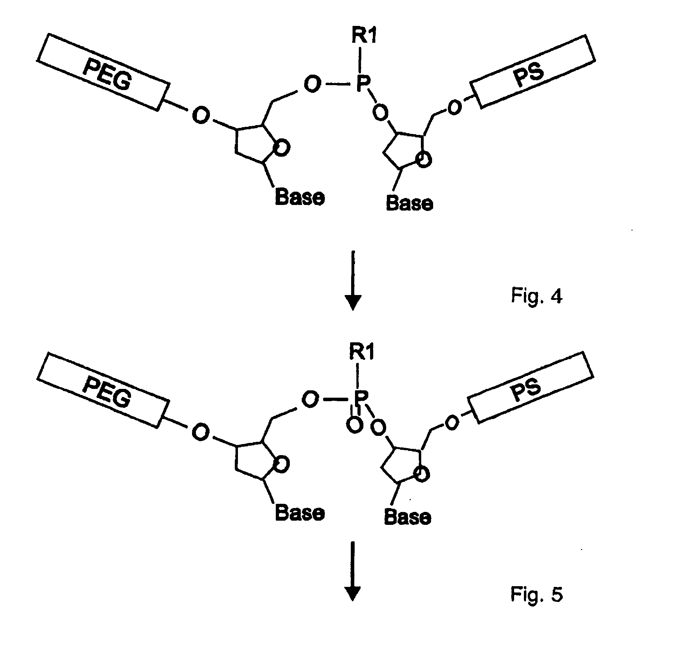

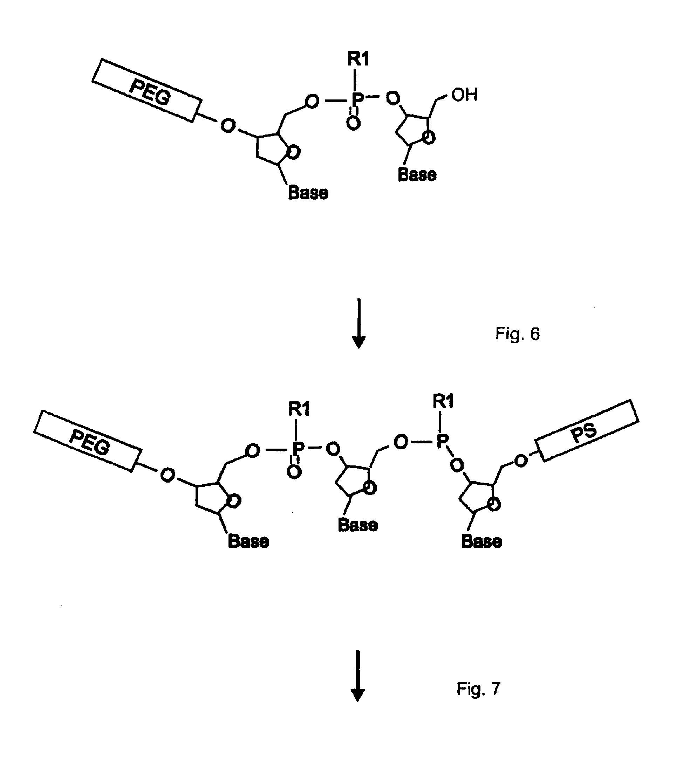

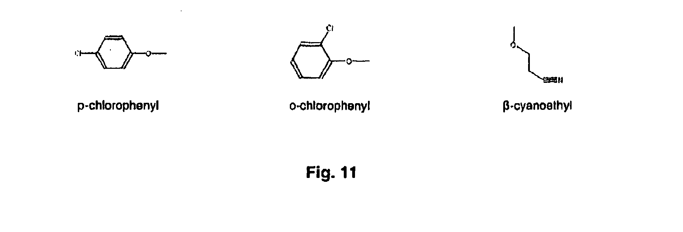

Nach Durchführung einer Fällungsreaktion und ggf. mindestens eines Waschschritts wird im nächsten in Fig. 3 dargestellten Verfahrensschritt die erste Verbindung mit einem Nukleosid-Phosphoramidit, an dessen 5'-Sauerstoffatom eine Polystyrol(PS)-Gruppe gebunden ist, als zweite Verbindung in Kontakt gebracht. Bei R1 handelt es sich um eine die Phosphatgruppe während der Flüssigphasensynthese vor nukleophilen Angriffen schützende Schutzgruppe. Die Schutzgruppe kann nach der Synthese, bevorzugt gemeinsam mit der Abspaltung der PEG- oder PS-Gruppe, abgespalten werden. Beispiele für derartige Schutzgruppen sind in Fig. 11 dargestellt. Bei R2 handelt es sich um einen Diisopropylamin-Rest. Die Phosphoramidit-Gruppe ist am 3'-Sauerstoffatom angeordnet. Die Phosphoramidit-Gruppe reagiert mit der freien OH-Gruppe des ersten Nukleotids in einer Kondensationsreaktion und führt zur Bildung eines in Fig. 4 dargestellten Dimers, bei welchem der Phosphor in Oxidationsstufe +3 vorliegt. Um beim späteren Abspalten des Polystyrols eine Spaltung am Phosphor zu verhindern, wird dieser durch Zugabe einer wässrigen Jodlösung auf die Oxidationsstufe +5 oxidiert. Das entsprechende Reaktionsprodukt ist in Fig. 5 dargestellt. Dieses Reaktionsprodukt kann bereits durch die Zugabe der wässrigen Jodlösung spezifisch ausfallen. Alternativ oder zusätzlich kann nicht gefälltes Reaktionsprodukt durch eine für Polystyrol spezifische Fällungsreaktion zusammen mit nicht umgesetzter zweiter Verbindung gefällt werden. Das Reaktionsprodukt wird, beispielsweise durch Zentrifugation, von ggf. noch vorhandener aber nicht umgesetzter erster Verbindung abgesondert und ggf. gewaschen. Nach Lösen des Reaktionsprodukts in einem Lösungsmittel, in welchem sich die zweite Verbindung nicht löst, wird eine Spaltungsreaktion durchgeführt, bei welcher das Polystyrol abgetrennt wird und am 5'-Ende des Dimers eine OH-Gruppe als aktive Gruppe entsteht. Das entsprechende Dimer ist in Fig. 6 dargestellt.

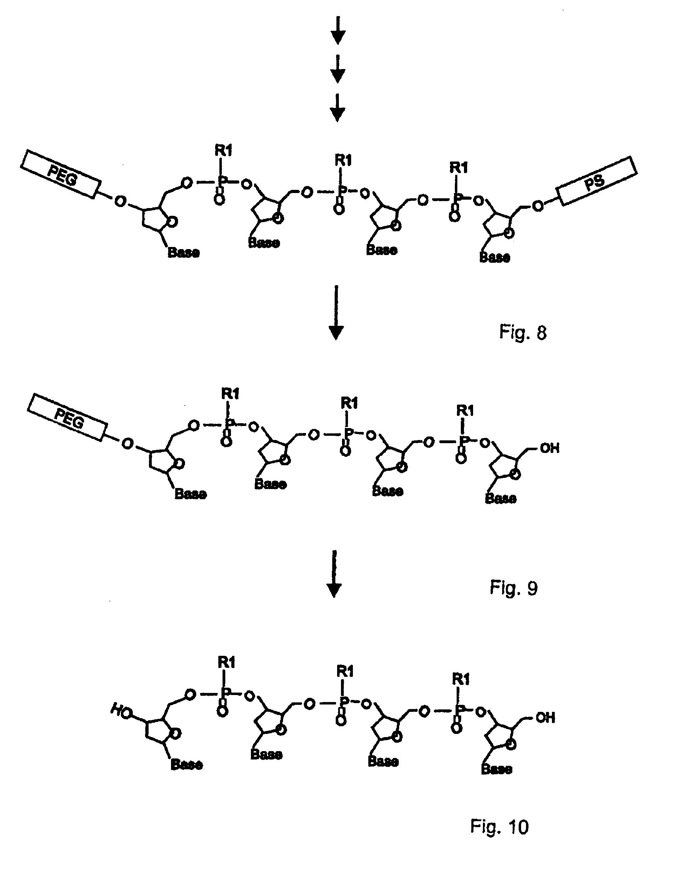

Nachfolgend wird dieses Dimer anstelle der ersten Verbindung mit einem weiteren Nukleosid-Phosphoramidit mit daran an dessen 5'-Ende gebundener Polystyrol-Gruppe als weitere Verbindung in Kontakt gebracht. Durch Kondensation entsteht das in Fig. 7 dargestellte Trimer. Nach Oxidation des in Oxidationsstufe +3 vorliegenden Phosphors auf die Oxidationsstufe +5, einer für Polystyrol spezifischen Fällung, erneutem Lösen des Trimers in einem Lösungsmittel, in welchem sich die weitere Verbindung nicht löst, und Abspalten des Polystyrols steht das Trimer für weitere Kondensationsschritte zur Verfügung, so dass ein Polymer spezifischer Länge gebildet werden kann. Als Beispiel dafür ist in Fig. 8 eine Tetramer dargestellt. Dieses Tetramer wird in einer für Polystyrol spezifischen Fällungsreaktion gefällt und wieder spezifisch gelöst. Das nach Abspaltung des Polystyrols verbleibende Reaktionsprodukt ist in Fig. 9 dargestellt. Es wird in einer für Polyethylenglycol spezifischen Fällungsreaktion gefällt, abgesondert und wieder gelöst. Anschließend wird in einer Spaltungsreaktion das Polyethylenglycol abgespalten und in einer für Polyethylenglycol spezifischen Fällungsreaktion vom in Lösung verbleibenden in Fig. 10 dargestellten Reaktionsprodukt (erstes Polymer) getrennt. Dieses kann anschließend durch eine Präzipitation, beispielsweise mittels Alkohol, ebenfalls gefällt werden.

## Patentansprüche

1. Verfahren zur Flüssigphasensynthese eines aus n Monomeren gebildeten ersten Polymers mit folgenden Schritten:
a) Bereitstellen einer gelösten ersten Verbindung, wobei die erste Verbindung aus einem ersten Monomer und einem eine erste spezifische Löslichkeit aufweisenden zweiten Polymer gebildet ist, wobei das erste Monomer und das zweite Polymer durch eine erste Bindung miteinander verbunden sind, wobei die erste Bindung durch eine erste Spaltungsreaktion gespalten werden kann, wobei das erste Monomer eine aktive oder eine aktivierbare erste Gruppe aufweist,
b) Bereitstellen einer gelösten zweiten Verbindung, wobei die zweite Verbindung aus einem zweiten Monomer und einem eine zweite spezifische Löslichkeit aufweisenden dritten Polymer gebildet ist, wobei das zweite Monomer und das dritte Polymer durch eine zweite Bindung miteinander verbunden sind, wobei die zweite Bindung durch eine zweite Spaltungsreaktion gespalten werden kann, wobei das zweite Monomer eine aktive oder eine aktivierbare zweite Gruppe aufweist, welche durch eine Verknüpfungsreaktion mit der ersten Gruppe unter Ausbildung einer dritten Bindung zwischen dem ersten und dem zweiten Monomer reagieren kann, wobei die erste und die zweite spezifische Löslichkeit verschieden sind,
c) Inkontaktbringen der ersten und der zweiten Verbindung, so dass die Verknüpfungsreaktion erfolgt, wobei im Falle des Vorliegens der aktivierbaren ersten und/oder der aktivierbaren zweiten Gruppe eine Aktivierung dieser ersten und/oder zweiten Gruppe durch Zusatz eines Aktivators erfolgt,
d) Durchführen einer für das zweite oder dritte Polymer spezifischen Fällungsreaktion und Absondern dabei gefällter erster oder zweiter Verbindung und des bei Schritt lit. c) gebildeten Reaktionsprodukts,
e1) Lösen der abgesonderten ersten oder zweiten Verbindung und des Reaktionsprodukts in einem Lösungsmittel, Durchführen einer für dasjenige zweite oder dritte Polymer spezifischen Fällungsreaktion für das die bei Schritt lit. d) durchgeführte Fällungsreaktion nicht spezifisch war, Absondern dabei gefällter Reaktionsprodukte und Lösen des Reaktionsprodukts in dem Lösungsmittel oder einem weiteren Lösungsmittel,
oder
e2) Inkontaktbringen der abgesonderten ersten oder zweiten Verbindung und des Reaktionsprodukts mit einem Lösungsmittel, in welchem sich im Wesentlichen nur das Reaktionsprodukt löst, in welchem sich aber im Wesentlichen nicht die bei Schritt lit. d) gefällte erste oder zweite Verbindung löst,
f) Durchführen der zweiten Spaltungsreaktion mit dem Reaktionsprodukt, wobei ein weiteres Reaktionsprodukt und das dritte Polymer gebildet werden, wobei an dem weiteren Reaktionsprodukt die erste aktive Gruppe gebildet wird und
g) (n-2)-faches Wiederholen der Schritte lit. b) bis lit. f), wobei an Stelle der ersten Verbindung das bei Schritt lit. f) gebildete weitere Reaktionsprodukt tritt und an Stelle der zweiten Verbindung jeweils eine weitere Verbindung tritt, wobei die weitere Verbindung aus einem weiteren Monomer und dem dritten Polymer gebildet ist,
wobei es sich bei dem ersten und dem zweiten Monomer jeweils um ein Nukleotid handelt.

2. Verfahren nach Anspruch 1, wobei nach Schritt lit. g) oder zwischen den Schritten lit. f) und lit. g)
eine für das zweite Polymer spezifische Fällung durchgeführt und das dabei gefällte, bei Schritt lit. f) gebildete weitere Reaktionsprodukt abgesondert wird
oder
eine für das dritte Polymer spezifische Fällung durchgeführt und das dabei gefällte, bei Schritt lit. f) gebildete dritte Polymer abgesondert wird.

3. Verfahren nach einem der vorhergehenden Ansprüche, wobei die aktivierbare erste Gruppe aus einem am 5'-C-Atom des Nukleotids gebundenen Sauerstoffatom und einer daran gebundenen Schutzgruppe, insbesondere einer Dimethoxytritylgruppe, dem dritten Polymer oder dem an eine Dimethoxytritylgruppe gebundenen dritten Polymer, besteht.

4. Verfahren nach Anspruch 3, wobei die Aktivierung der aktivierbaren ersten Gruppe durch eine Abspaltung der Schutzgruppe unter Reduktion des Sauerstoffatoms, insbesondere durch eine organische Säure als Aktivator, erfolgt.

5. Verfahren nach einem der vorhergehenden Ansprüche, wobei die aktive erste Gruppe eine am 5'-C-Atom des das erste Monomer bildenden Nukleotids angeordnete OH-Gruppe ist.

6. Verfahren nach einem der vorhergehenden Ansprüche, wobei das zweite Monomer ein Nukleosid-Phosphoramidit ist.

7. Verfahren nach Anspruch 6, wobei das Nukleosid-Phosphoramidit vor dem oder beim Inkontaktbringen gemäß Schritt lit. c), insbesondere mittels H-Tetrazol, Ethylthiotetrazol oder einem anderen Tetrazolderivat oder Dicyanoimidazol, durch Protonierung aktiviert wird.

8. Verfahren nach Anspruch 6 oder 7, wobei zwischen den Schritten lit. c) und lit. g) ein Oxidationsschritt zur Oxidation des gebildeten Phosphittriesters zum Phosphotriester, insbesondere mittels Jod als Oxidationsmittel, durchgeführt wird.

9. Verfahren nach einem der vorhergehenden Ansprüche, wobei das erste Monomer über ein Sauerstoffatom, insbesondere innerhalb einer Etherbindung oder einer Esterbindung, an das zweite Polymer gebunden ist.

10. Verfahren nach einem der vorhergehenden Ansprüche, wobei nach dem (n-2)-fachen Wiederholen der Schritte lit. b) bis lit. f) gemäß Schritt lit. g) die erste Spaltungsreaktion durchgeführt wird.

11. Verfahren nach Anspruch 10, wobei bei Durchführung der ersten Spaltungsreaktion eine Reduktion des gemäß Anspruch 8 gebildeten Phosphotriesters erfolgt.

12. Verfahren nach Anspruch 10 oder 11, wobei die erste Spaltungsreaktion mittels einer Ammoniumhydroxid-Lösung durchgeführt wird.

13. Verfahren nach einem der vorhergehenden Ansprüche, wobei das zweite oder das dritte Polymer Polyethylenglycol ist, wobei das zweite und das dritte Polymer stets verschieden sind.

14. Verfahren nach einem der vorhergehenden Ansprüche, wobei das dritte oder das zweite Polymer Polystyrol ist, wobei das zweite und das dritte Polymer stets verschieden sind.

## Claims

1. Method for fluid-phase synthesis of a first polymer formed from n monomers, comprising the following steps:
a) providing a dissolved first compound, wherein said first compound is formed from a first monomer and a second polymer having a first specific solubility, wherein said first monomer and said second polymer are linked to one another by a first bond, wherein said first bond can be cleaved by a first cleavage reaction, wherein said first monomer has an active or an activatable first group,
b) providing a dissolved second compound, wherein said second compound is formed from a second monomer and a third polymer having a second specific solubility, wherein said second monomer and said third polymer are linked to one another by a second bond, wherein said second bond can be cleaved by a second cleavage reaction, wherein said second monomer has an active or an activatable second group which, by way of a linking reaction, can react with the first group with formation of a third bond between the first and second monomers, wherein said first and second specific solubilities are different,
c) contacting the first and second compounds in order for the linking reaction to occur, wherein, if the activatable first and/or the activatable second group are/is present, said first and/or said second group are/is activated by adding an activator,
d) carrying out a precipitation reaction specific for the second or third polymer, and removing any first or second compound precipitated in the process and the reaction product formed in step c),
e1) dissolving the removed first or second compound and the reaction product in a solvent, carrying out a precipitation reaction specific for the second or third polymer for which the precipitation reaction carried out in step d) was not specific, removing reaction products precipitated in the process, and dissolving the reaction product in said solvent or another solvent,
or
e2) contacting the removed first or second compound and the reaction product with a solvent in which essentially only the reaction product dissolves but in which the first or second compound precipitated in step d) essentially does not dissolve,
f) carrying out the second cleavage reaction with the reaction product, wherein a further reaction product and the third polymer are formed, wherein the first active group is formed on said further reaction product, and
g) (n-2)-fold repetition of steps b) to f), wherein the first compound is replaced by the further reaction product formed in step f), and the second compound is replaced in each case with a further compound, wherein said further compound is formed from a further monomer and the third polymer,
wherein both the first and second monomers are nucleotides.

2. Method according to Claim 1, wherein a precipitation specific for the second polymer is carried out after step g) or between steps f) and g), and the further reaction product precipitated in the process and formed in step f) is removed,
or
a precipitation specific for the third polymer is carried out after step g) or between steps f) and g), and the third polymer precipitated in the process and formed in step f) is removed.

3. Method according to either of the preceding claims, wherein the activatable first group consists of an oxygen atom bound to the 5' carbon atom of the nucleotide and a protective group, in particular a dimethoxytrityl group, the third polymer or the third polymer bound to a dimethoxytrityl group, which protective group is bound to said oxygen atom.

4. Method according to Claim 3, wherein the activatable first group is activated by cleaving off the protective group with reduction of the oxygen atom, in particular by means of an organic acid as activator.

5. Method according to any of the preceding claims, wherein the active first group is an OH group arranged on the 5' carbon atom of the nucleotide forming the first monomer.

6. Method according to any of the preceding claims, wherein the second monomer is a nucleoside phosphoramidite.

7. Method according to Claim 6, wherein the nucleoside phosphoramidite is activated by protonation, in particular by means of H-tetrazole, ethylthiotetrazole or another tetrazole derivative or dicyanoimidazole, prior to or during contacting according to step c).

8. Method according to Claim 6 or 7, wherein an oxidation step is carried out between steps c) and g) in order to oxidize the phosphite triester formed to a phosphotriester, in particular by means of iodine as oxidant.

9. Method according to any of the preceding claims, wherein the first monomer is bound via an oxygen atom, in particular within an ether bond or an ester bond, to the second polymer.

10. Method according to any of the preceding claims, wherein the first cleavage reaction is carried out after repeating steps b) to f) (n-2) times, according to step g).

11. Method according to Claim 10, wherein the phosphotriester formed according to Claim 8 is reduced in the course of the first cleavage reaction.

12. Method according to Claim 10 or 11, wherein the first cleavage reaction is carried out by means of an ammonium hydroxide solution.

13. Method according to any of the preceding claims, wherein the second or the third polymer is polyethylene glycol, wherein the second and third polymers are always different.

14. Method according to any of the preceding claims, wherein the third or the second polymer is polystyrene, wherein the second and third polymers are always different.

## Revendications

1. Procédé de synthèse en phase liquide d'un premier polymère formé à partir de n monomères, comprenant les étapes suivantes :
a) mise à disposition d'un premier composé dissous, où le premier composé est formé d'un premier monomère et d'un second polymère présentant une première solubilité spécifique, où le premier monomère et le second polymère sont reliés ensemble par une première liaison, où la première liaison peut être séparée par une première réaction de scission, où le premier monomère présente un premier groupe actif ou un premier groupe activable,
b) mise à disposition d'un second composé dissous, où le second composé est formé d'un second monomère et d'un troisième polymère présentant une seconde solubilité spécifique, où le second monomère et le troisième polymère sont reliés ensemble par une seconde liaison, où la seconde liaison peut être séparée par une seconde réaction de scission, où le second monomère présente un second groupe actif ou un second groupe activable, qui peut réagir par une réaction de réticulation avec le premier groupe en formant une troisième liaison entre le premier et le second monomère, où la première et la seconde solubilité sont différentes ;
c) mise en contact du premier et du second composé de façon telle que la réaction de réticulation se produit, où en cas de présence du premier groupe activable et/ou du second groupe activable se produit une activation de ce premier et/ou second groupe par ajout d'un activateur,
d) réalisation d'une réaction de précipitation spécifique du second ou du troisième polymère et séparation du premier ou second composé alors précipité et du produit réactionnel formé lors de l'étape c),
e1) dissolution du premier ou du second composé séparé et du produit réactionnel dans un solvant, réalisation d'une réaction de précipitation spécifique du second ou du troisième polymère pour lequel la réaction de précipitation menée lors de l'étape d) n'était pas spécifique, séparation des produits réactionnels alors précipités et dissolution du produit réactionnel dans le solvant ou dans un autre solvant,
ou
e2) mise en contact du premier ou du second composé séparé et du produit réactionnel avec un solvant dans lequel se dissout essentiellement uniquement le produit réactionnel, dans lequel par contre ne se dissout essentiellement pas le premier ou le second composé précipité lors de l'étape d),
f) réalisation de la seconde réaction de scission avec le produit réactionnel, où un autre produit réactionnel et le troisième polymère sont formés, où le premier groupe actif est formé au niveau de l'autre produit réactionnel, et
g) répétition (n-2) fois des étapes b) à f), où à la place du premier composé, intervient l'autre produit réactionnel formé lors de l'étape f) et à la place du second composé intervient respectivement un autre composé, où l'autre composé est formé d'un autre monomère et du troisième polymère,
où il s'agit concernant le premier et le second monomère respectivement d'un nucléotide.

2. Procédé selon la revendication 1, dans lequel après l'étape g) ou entre les étapes f) et g)
on réalise une précipitation spécifique du second polymère et on sépare l'autre produit réactionnel alors précipité formé lors de l'étape f)
ou
on réalise une précipitation spécifique du troisième polymère et on sépare le troisième polymère alors précipité formé lors de l'étape f).

3. Procédé selon l'une des revendications précédentes, dans lequel le premier groupe activable se compose d'un atome d'oxygène lié à l'atome 5'-C du nucléotide et d'un groupe protecteur qui y est lié, en particulier d'un groupe diméthoxytrityle, du troisième polymère ou du troisième polymère lié à un groupe diméthoxytrityle.

4. Procédé selon la revendication 3, dans lequel l'activation du premier groupe activable se fait par une scission du groupe protecteur avec réduction de l'atome d'oxygène, en particulier par un acide organique en tant qu'activateur.

5. Procédé selon l'une des revendications précédentes, dans lequel le premier groupe actif est un groupe OH disposé au niveau de l'atome 5'-C du nucléotide formant le premier monomère.

6. Procédé selon l'une des revendications précédentes, dans lequel le second monomère est un nucléoside-phosphoramidite.

7. Procédé selon la revendication 6, dans lequel le nucléoside-phosphoramidite est activé par protonation avant ou lors de la mise en contact selon l'étape c), en particulier au moyen d'un H-tétrazole, d'un éthylthiotétrazole ou d'un autre dérivé tétrazole ou d'un dicyanoimidazole.

8. Procédé selon la revendication 6 ou 7, dans lequel entre les étapes c) et g) est réalisée une étape d'oxydation permettant l'oxydation du triester de phosphite formé en phosphotriester, en particulier au moyen d'iode en tant qu'agent oxydant.

9. Procédé selon l'une des revendications précédentes, dans lequel le premier monomère est lié au second polymère par un atome d'oxygène, en particulier au sein d'une liaison éther ou d'une liaison ester.

10. Procédé selon l'une des revendications précédentes, dans lequel après la répétition (n-2) fois des étapes b) à f), on réalise la première réaction de scission selon l'étape g).

11. Procédé selon la revendication 10, dans lequel lors de la réalisation de la première réaction de scission se produit une réduction du phosphotriester formé selon la revendication 8.

12. Procédé selon la revendication 10 ou 11, dans lequel la première réaction de scission est réalisée au moyen d'une solution d'hydroxyde d'ammonium.

13. Procédé selon l'une des revendications précédentes, dans lequel le second ou le troisième polymère est un polyéthylèneglycol, le second et le troisième polymères étant toujours différents.

14. Procédé selon l'une des revendications précédentes, dans lequel le troisième ou le second polymère est un polystyrène, le second et le troisième polymères étant toujours différents.
